# EUROPEAN PATENT APPLICATION

(11) **EP 4 527 444 A1**
(43) Date of publication of application: **26.03.2025**
(21) Application number: 23827377.5
(22) Date of filing: 16.05.2023
(51) Int. Cl.: A61N 1/36, A61N 1/04

(54) **ELECTRICAL STIMULATION THERAPY APPARATUS FOR SLEEP APNEA, AND OPERATING METHOD THEREFOR**

(30) Priority: 20.06.2022 KR 20220074627
(71) Applicant: Seoul National University R & DB Foundation, Seoul 08826 (KR); RS Rehab Co., Ltd., Seongnam-si, Gyeonggi-do 13449 (KR)
(72) Inventor: RYU, Ju Seok, Seongnam-si Gyeonggi-do 13475 (KR); LEE, Jun Chang, Seoul 02595 (KR)
(74) Representative: Manitz Finsterwald Patent- und Rechtsanwaltspartnerschaft mbB
(86) International application number: PCT/KR2023/006604
(87) International publication number: WO 2023/249257

(57) **Abstract**

According to an embodiment of the present disclosure, an electrical stimulation therapy apparatus for sleep apnea includes: a plurality of stimulation units attached to muscles involved in sleep apnea and configured with three or more channels; and a processor which generates a control command to drive each of the stimulation units, and the processor uses the stimulation units to sequentially stimulate a tongue base, a thyrohyoid muscle, and an infrahyoid muscle of a user according to a preset muscle activation sequence.

## Description

### TECHNICAL FIELD

The present disclosure relates to an electrical stimulation therapy apparatus for sleep apnea and an operation method thereof.

### BACKGROUND

Sleep apnea is a condition in which the muscles that support the soft tissues of the throat, tongue, and soft palate relax and the airway becomes narrow, leading to pauses in breathing. There are three types of sleep apnea: obstructive sleep apnea, central sleep apnea, and mixed sleep apnea. Obstructive sleep apnea is the most common type of sleep apnea, while central sleep apnea is a much less common type than obstructive sleep apnea. Mixed sleep apnea is a combination of the two other types of sleep apnea.

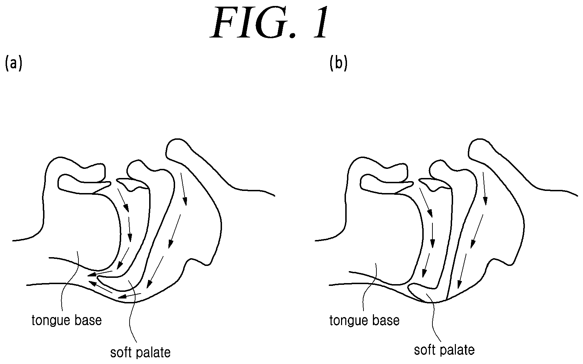

**FIG. 1** is a diagram provided to describe symptoms of obstructive sleep apnea. **FIG. 1(a)** shows a normal state, and **FIG. 1(b)** shows the airway being obstructed due to backward collapse of the soft palate and tongue base. Obstructive sleep apnea occurs when tension in the tongue base including the soft palate and the genioglossus muscle decreases and the tongue base and the soft palate are pushed backwards and come into contact with the posterior pharyngeal wall during sleep. That is, obstructive sleep apnea is a condition in which the airway becomes blocked as the tension in the tongue base decreases, leading to pauses in breathing.

Conventional therapies for sleep apnea are based on the fact that the genioglossus and the upper and middle pharyngeal constrictor muscles are involved in maintaining the airway in the pharynx. Accordingly, a therapy that stimulates the hypoglossal nerve is being researched to improve airway maintenance in the pharynx.

In this regard, U.S. Patent No. 8774943 (Title of Invention: Apparatus and method for treating obstructive sleep apnea) discloses an apparatus and operation method for treating obstructive sleep apnea by monitoring electrical stimulation of the hypoglossal nerve with a dental fixture.

### DISCLOSURE OF THE INVENTION

### PROBLEMS TO BE SOLVED BY THE INVENTION

Some embodiments of the present disclosure are conceived to provide an electrical stimulation therapy apparatus and method for sleep apnea, which applies electrical stimulation to the muscles involved in the occurrence of obstructive sleep apnea according to a preset muscle activation sequence.

The problems to be solved by the present disclosure are not limited to the above-described problems. There may be other problems to be solved by the present disclosure.

### MEANS FOR SOLVING THE PROBLEMS

According to an aspect of the present disclosure, an electrical stimulation therapy apparatus for sleep apnea includes: a plurality of stimulation units attached to muscles involved in sleep apnea and configured with three or more channels; and a processor which generates a control command to drive each of the stimulation units, and the processor uses the stimulation units to sequentially stimulate a tongue base, a thyrohyoid muscle, and an infrahyoid muscle of a user according to a preset muscle activation sequence.

According to another aspect of the present disclosure, an operation method of an electrical stimulation therapy apparatus for sleep apnea includes: (a) attaching a plurality of stimulation units, which is configured with three or more channels, to muscles involved in sleep apnea; and (b) sequentially stimulating a tongue base, a thyrohyoid muscle, and an infrahyoid muscle of a user according to a preset muscle activation sequence by using the stimulation units.

### EFFECTS OF THE INVENTION

According to an embodiment of the present disclosure, it is possible to sequentially apply electrical stimulation to muscles involved in the occurrence of obstructive sleep apnea by using a plurality of stimulation units, which is configured with three or more channels. Thus, it is possible to strengthen the soft palate and the tongue base of the user and improve the tension.

### BRIEF DESCRIPTION OF THE DRAWINGS

**FIG. 1** is a diagram provided to describe symptoms of obstructive sleep apnea.
**FIG. 2** is a configuration diagram of an electrical stimulation therapy apparatus for sleep apnea according to an embodiment of the present disclosure.
**FIG. 3** illustrates the tongue base involved in sleep apnea.
**FIG. 4** and **FIG. 5** illustrate a configuration of stimulation units according to an embodiment of the present disclosure.
**FIG. 6** and **FIG. 7** illustrate a configuration of stimulation units according to another embodiment of the present disclosure.
**FIG. 8** and **FIG. 9** show examples of displacement of muscles involved in sleep apnea depending on electrical stimulation of the electrical stimulation therapy apparatus for sleep apnea according to an embodiment of the present disclosure.
**FIG. 10** is a graph for describing the result of a test to compare oxygen saturation levels of a user by using the electrical stimulation therapy apparatus for sleep apnea according to an embodiment of the present disclosure.
**FIG. 11** is a flowchart showing an operation method of an electrical stimulation therapy apparatus for sleep apnea according to an embodiment of the present disclosure.

### BEST MODE FOR CARRYING OUT THE INVENTION

Hereafter, embodiments will be described in detail with reference to the accompanying drawings so that the present disclosure may be readily implemented by a person with ordinary skill in the art. However, it is to be noted that the present disclosure is not limited to the embodiments but can be embodied in various other ways. In the drawings, parts irrelevant to the description are omitted for the simplicity of explanation, and like reference numerals denote like parts through the whole document.

Throughout the whole document, the term "connected to" or "coupled to" that is used to designate a connection or coupling of one element to another element includes both a case that an element is "directly connected or coupled to" another element and a case that an element is "electronically connected or coupled to" another element via still another element. Further, it is to be understood that the term "comprises or includes" and/or "comprising or including" used in the document means that one or more other components, steps, operation and/or existence or addition of elements are not excluded in addition to the described components, steps, operation and/or elements unless context dictates otherwise and is not intended to preclude the possibility that one or more other features, numbers, steps, operations, components, parts, or combinations thereof may exist or may be added.

Throughout the whole document, the term "unit" includes a unit implemented by hardware or software and a unit implemented by both of them. One unit may be implemented by two or more pieces of hardware, and two or more units may be implemented by one piece of hardware. However, the "unit" is not limited to the software or the hardware and may be stored in an addressable storage medium or may be configured to implement one or more processors. Accordingly, the "unit" may include, for example, software, object-oriented software, classes, tasks, processes, functions, attributes, procedures, sub-routines, segments of program codes, drivers, firmware, micro codes, circuits, data, database, data structures, tables, arrays, variables and the like. The functions provided in the components and the "units" may be combined into a smaller number of components and "units" or may be further separated into additional components and "units". Further, the components and the "units" may also be implemented to play one or more central processing units (CPUs) in a device.

The term "network" refers to a connection structure that enables information exchange between nodes such as devices, servers, etc. and includes LAN (Local Area Network), WAN (Wide Area Network), Internet (WWW: World Wide Web), a wired or wireless data communication network, a telecommunication network, a wired or wireless television network, and the like. Examples of the wireless data communication network may include 3G, 4G, 5G, 3GPP (3rd Generation Partnership Project), LTE (Long Term Evolution), WIMAX (World Interoperability for Microwave Access), Wi-Fi, Bluetooth communication, infrared communication, ultrasonic communication, VLC (Visible Light Communication), LiFi, and the like, but may not be limited thereto.

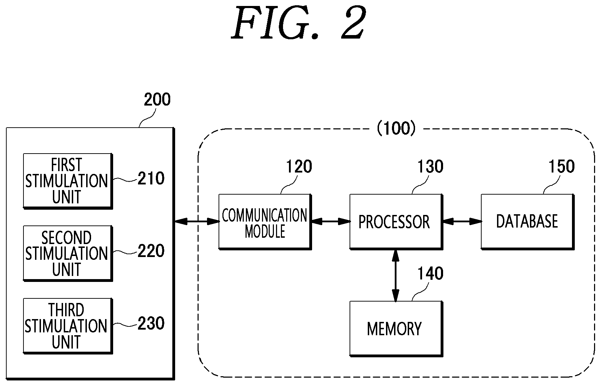

**FIG. 2** is a configuration diagram of an electrical stimulation therapy apparatus for sleep apnea according to an embodiment of the present disclosure.

As shown in **FIG. 2****,** an electrical stimulation therapy apparatus 100 for sleep apnea includes a plurality of stimulation units 200 attached to muscles involved in sleep apnea and configured with three or more channels, and a processor 130 which generates a control command to drive each of the stimulation units 200, and the processor 130 uses the stimulation units 200 to sequentially stimulate a tongue base, a thyrohyoid muscle, and an infrahyoid muscle of a user according to a preset muscle activation sequence.

Therefore, according to the present disclosure, it is possible to sequentially apply electrical stimulation to muscles involved in the occurrence of obstructive sleep apnea by using a plurality of stimulation units, which is configured with three or more channels. Thus, it is possible to strengthen the soft palate and the tongue base of the user and improve the tension.

Specifically, as illustrated in **FIG. 2****,** the electrical stimulation therapy apparatus 100 includes a communication module 120, the processor 130, a memory 140, and the stimulation units 200.

The communication module 120 performs data communication with each of the stimulation units 200. The communication module 120 provides a communication interface necessary to provide signals transmitted to and received from respective units through a communication network in the form of packet data. Here, the communication module 120 may include hardware and software necessary for transmitting and receiving signals such as control signals or data signals through wired/wireless connection with other network devices.

The memory 140 may store a program for controlling the stimulation unit, and the program for controlling the stimulation units stored in the memory may be driven by the processor 130.

Further, the memory 140 performs a function of temporarily or permanently storing data processed by the processor 130. Here, the memory 140 may include a volatile storage medium or a non-volatile storage medium, but the scope of the present disclosure is not limited thereto.

The memory 140 may store a separate program such as an operating system for processing and controlling the processor 130 or may perform a function of temporarily storing input data or output data.

The memory 140 may include at least one type of storage medium among a flash memory type memory, a hard disk type memory, a multimedia card micro type memory, a card type memory (for example, a secure digital (SD) memory or an extreme digital (XD) memory), a random access memory (RAM), and a read-only memory (ROM). Also, a user device may operate a web storage that performs a storage function of the memory 140 on the Internet.

The processor 130 executes a program for controlling the stimulation units stored in the memory 140 and controls the overall operation for controlling the electrical stimulation therapy apparatus for sleep apnea.

To this end, the processor 130 may include one or more processing units (a central processing unit (CPU), a micro-processor, a digital signal processor (DSP), etc.), RAM, ROM, and the like, and may read a program stored in the memory 140 into the RAM and execute the program by using the one or more processing units. Further, according to an embodiment, the term "processor" may be interpreted as the same meaning as the terms such as "controller", "arithmetic unit", or "control unit".

A schematic procedure of controlling an electrical stimulation therapy apparatus for sleep apnea through a program for controlling the stimulation units according to an embodiment of the present disclosure is as follows.

The processor 130 may control the stimulation units 200 such that muscles involved in sleep apnea and including the tongue base, the thyrohyoid muscle, and the infrahyoid muscle are sequentially stimulated according to the preset muscle activation sequence upon execution of the program.

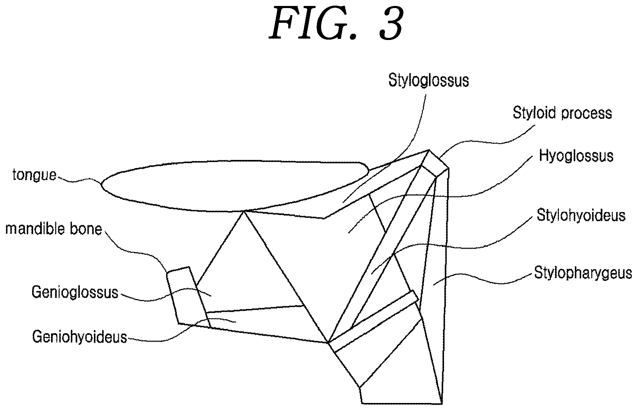

**FIG. 3** illustrates the tongue base involved in sleep apnea.

Referring back to **FIG. 1B****,** sleep apnea is a condition in which the soft palate and the tongue base decrease in tension and fall backwards and thus block the airway.

As shown in **FIG. 3****,** the muscles of the tongue base include the genioglossus muscle, the geniohyoid muscle, and the hyoglossus muscle. In particular, the genioglossus muscle is mainly involved in sleep apnea. Herein, the styloglossus muscle, the stylohyoid muscle, and the stylopharyngeus muscle are involved in fixing the muscles of the tongue base to the styloid process.

Also, the muscles anatomically involved in sleep apnea are primarily located in the deep part of the anterior neck. Therefore, the stimulation units 200 can be attached to the front part of the neck to stimulate the muscles involved in sleep apnea. The front part of the neck can be broadly divided into the suprahyoid muscle and the infrahyoid muscle based on the hyoid bone.

First, the processor 130 can collect and record the muscle activation sequence in advance by using electrodes of channels attached to the muscles involved in sleep apnea. Herein, the muscle activation sequence refers to an algorithm of sequential muscle contractions that can improve muscle strength and coordination through functional movements such as swallowing.

That is, the processor 130 can control the stimulation units 200 to sequentially stimulate the user's tongue base (the suprahyoid muscle), the thyrohyoid muscle, and the infrahyoid muscle based on the collected muscle activation sequence.

In other words, the electrical stimulation therapy apparatus for sleep apnea according to the present disclosure sequentially applies electrical stimulation to the muscles involved in the occurrence of obstructive sleep apnea by using the stimulation units 200. This increases the volume and tension of the muscles and can improve the symptoms of obstructive sleep apnea.

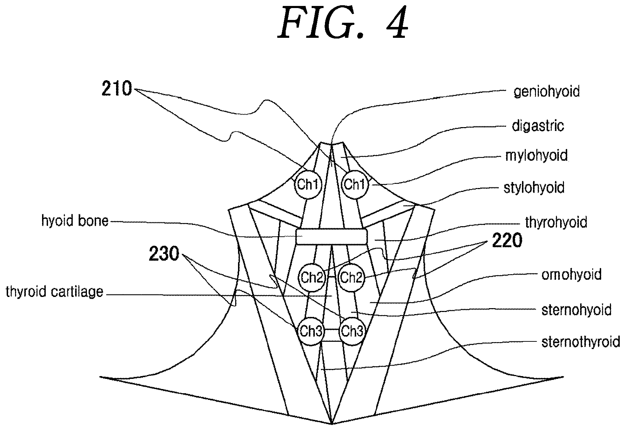

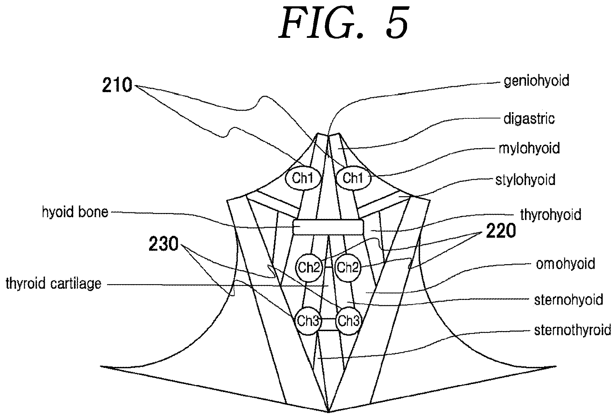

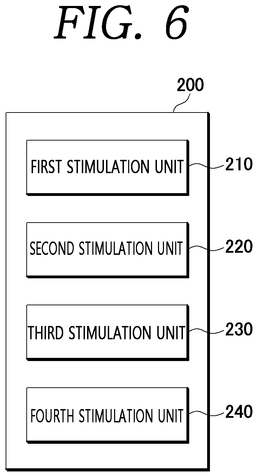

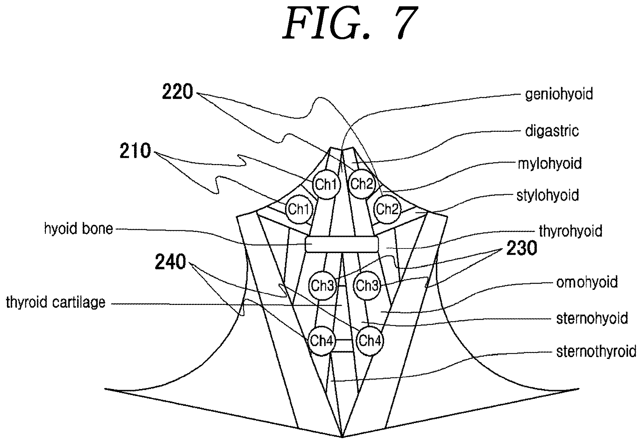

Hereafter, a configuration of the stimulation units 200 will be described with reference to **FIG. 4** to **FIG. 7****.** **FIG. 4** and **FIG. 5** illustrate a configuration of stimulation units according to an embodiment of the present disclosure, and **FIG. 6** and **FIG. 7** illustrate a configuration of stimulation units according to another embodiment of the present disclosure.

As shown in **FIG. 4** and **FIG. 5****,** the stimulation units 200 include: a first stimulation unit 210 attached to the suprahyoid muscle including the digastric muscle and the mylohyoid muscle to stimulate the tongue base including the genioglossus muscle, the geniohyoid muscle, and the hyoglossus muscle; a second stimulation unit 220 attached to the upper part of the thyroid cartilage to stimulate the thyrohyoid muscle; and a third stimulation unit 230 attached to the inner part of the sternocleidomastoid muscle and to the lower part of the thyroid cartilage to stimulate the infrahyoid muscle including the sternohyoid muscle, the sternothyroid muscle, and the omohyoid muscle.

For example, the stimulation units 200 may be configured with three channels or four channels. For example, the channel means a path through which a current flows when a closed circuit including a power source is formed and a current flows through the closed circuit. That is, the closed circuit may include a power source, two electrodes (an anode and a cathode) connected to the power source, and a muscle connecting the electrodes to each other and operate as one channel. In this case, the channel serves to apply electrical stimulation, and each channel may include two electrodes (a cathode and an anode).

As shown in **FIG. 4** and **FIG. 5****,** the stimulation units 200 may be configured with, for example, three channels. The stimulation units 200 may include the first stimulation unit 210 that forms a first channel Ch1, the second stimulation unit 220 that forms a second channel Ch2, and the third stimulation unit 230 that forms a third channel Ch3.

For example, the first stimulation unit 210 may include an electrode that is attached to each of the left and right sides of the digastric muscle and the mylohyoid muscle (the suprahyoid muscle) and forms the first channel Ch1. Herein, each channel of the first stimulation unit 210 forming the first channel Ch1 may be placed on an overlap portion of the digastric muscle and the mylohyoid muscle.

Further, as shown in **FIG. 5****,** each electrode of the first channel Ch1 may have a greater area than an electrode of the second channel Ch2 and the third channel Ch3 so as to cover the two muscles (the digastric muscle and the mylohyoid muscle). That is, the first stimulation unit 210 may apply electrical stimulation to the digastric muscle and the mylohyoid muscle as targets to stimulate the tongue base including the geniohyoid muscle, the genioglossus muscle, and the hyoglossus muscle.

Likewise, the second stimulation unit 220 may include an electrode that is attached to each of the upper parts of the left and right sides of the thyroid cartilage and forms the second channel Ch2, and the third stimulation unit 230 may include an electrode that is attached to each of the inner parts of the left and right sides of the sternocleidomastoid muscle and forms the third channel Ch3. That is, the second stimulation unit 220 may apply electrical stimulation to the upper part of the thyroid cartilage to stimulate the thyrohyoid muscle, and the third stimulation unit 230 may apply electrical stimulation to the inner part of the sternocleidomastoid muscle and the lower part of the thyroid cartilage to stimulate the infrahyoid muscle including the sternohyoid muscle, the sternothyroid muscle, and the omohyoid muscle.

Herein, the two electrodes may be spaced apart from each other by about 1 cm, but the present disclosure is not limited thereto. For example, if the two electrodes are located too close to each other, the entire area is stimulated, and as the distance between the electrodes increases, the contraction intensity may also increase.

Also, each stimulation unit 200 may have a frequency of 30 Hz to 100 Hz. In general, when the stimulation units 200 has a lower frequency, electrical stimulation is applied to a deeper portion.

As another example, referring to **FIG. 6****,** the stimulation units 200 may be configured with four channels. The stimulation units 200 include the first stimulation unit 210 forming the first channel Ch1, the second stimulation unit 220 forming the second channel Ch2, the third stimulation unit 230 forming the third channel Ch3, and a fourth stimulation unit 240 forming a fourth channel Ch4.

For example, referring to **FIG. 7****,** the first stimulation unit 210 includes an electrode that is attached to each of the left side of the digastric muscle and the mylohyoid muscle and forms the first channel Ch1, and the second stimulation unit 220 includes an electrode that is attached to each of the right side of the digastric muscle and the mylohyoid muscle and forms the second channel Ch2. Further, the third stimulation unit 230 includes an electrode that is attached to each of the upper parts of the left and right sides of the thyroid cartilage and forms the third channel Ch3, and the fourth stimulation unit 240 includes an electrode that is attached to each of the inner parts of the left and right sides of the sternocleidomastoid muscle and forms the fourth channel Ch4.

In other words, the processor 130 can control each of the three or four channels to sequentially stimulate the user's tongue base (the suprahyoid muscle), the thyrohyoid muscle, and the infrahyoid muscles.

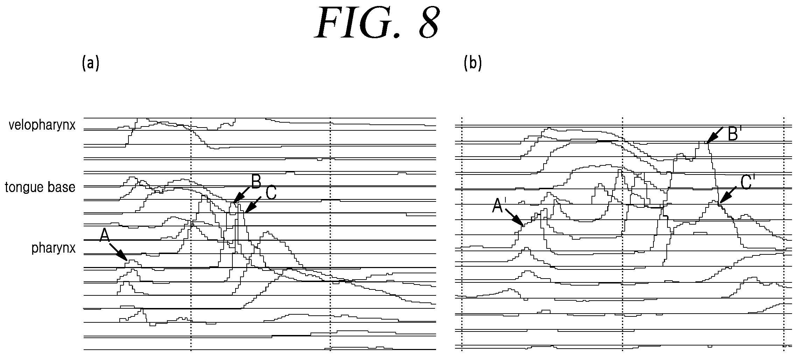

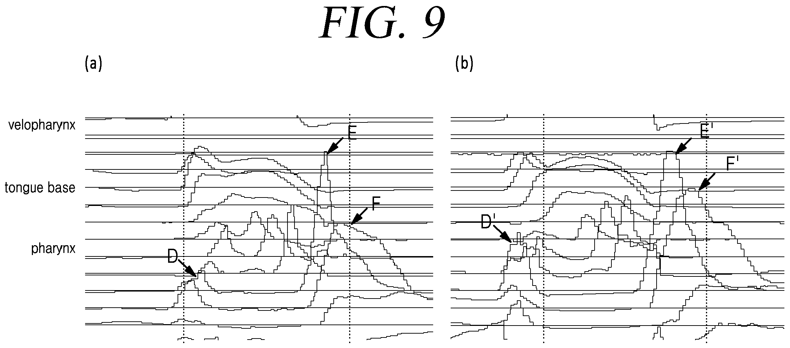

**FIG. 8** and **FIG. 9** show examples of displacement of muscles involved in sleep apnea depending on electrical stimulation of the electrical stimulation therapy apparatus for sleep apnea according to an embodiment of the present disclosure.

**FIG. 8** and **FIG. 9** show the measurement results from a videofluoroscopic swallowing study and a high-resolution esophageal manometry test performed while electrical stimulation is applied by using electrical stimulation therapy apparatus for sleep apnea according to the present disclosure and the user swallows.

**FIG. 8(a)** shows a pressure when the user swallows water in the absence of electrical stimulation, and **FIG. 8(b)** shows a pressure when the user swallows water in the presence of electrical stimulation. That is, as a result of measuring a functional movement such as swallowing of a low-viscosity liquid (*e.g*., water), it was confirmed that a contraction pressure and a contraction area of the tongue base (B', C') and the pharynx muscle (A') applied with electrical stimulation by the stimulation units 200 increased, compared to those of the tongue base (B, C) and the pharynx muscle (A) not applied with electrical stimulation.

**FIG. 9(a)** shows a pressure when the user swallows yogurt in the absence of electrical stimulation, and **FIG. 9(b)** shows a pressure when the user swallows yogurt in the presence of electrical stimulation. Similarly, as a result of measuring a functional movement such as swallowing of a high-viscosity liquid (*e.g*., yogurt), it was confirmed that a contraction pressure and a contraction area of the tongue base (E', F') and the pharynx muscle (D') applied with electrical stimulation by the stimulation units 200 increased, compared to those of the tongue base (E, F) and the pharynx muscle (D) not applied with electrical stimulation.

In summary, it was confirmed that when electrical stimulation was applied, a pressure measured from the velopharynx where the soft palate is located and the tongue base increased by about 30%. Therefore, it can be seen that a current from the electrical stimulation therapy apparatus for sleep apnea according to the present disclosure is transferred to the muscles involved in the occurrence of obstructive sleep apnea.

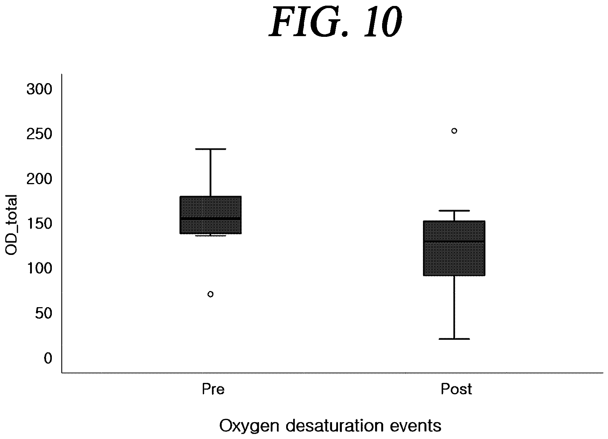

**FIG. 10** is a graph for describing the result of a test to compare oxygen saturation levels of a user by using the electrical stimulation therapy apparatus for sleep apnea according to an embodiment of the present disclosure.

**FIG. 10** shows that the number of oxygen desaturation events where the user's oxygen saturation levels temporarily drop to below 90% decreases after using the electrical stimulation therapy apparatus 100 for sleep apnea (Post) compared to before using it (Pre).

Hereinafter, descriptions of components performing the same function among the components described above with reference to **FIG. 1** to **FIG. 10** are omitted.

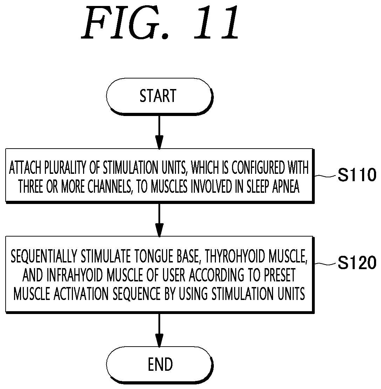

**FIG. 11** is a flowchart showing an operation method of an electrical stimulation therapy apparatus for sleep apnea according to an embodiment of the present disclosure.

Referring to **FIG. 11****,** an operation method of an electrical stimulation therapy apparatus for sleep apnea according to the present disclosure includes a process of attaching the plurality of stimulation units 200, which is configured with three or more channels, to muscles involved in sleep apnea (S110), and a process of sequentially stimulating a tongue base, a thyrohyoid muscle, and an infrahyoid muscle of a user according to a preset muscle activation sequence by using the stimulation units 200 (S120).

The stimulation units 200 include: the first stimulation unit 210 attached to the suprahyoid muscle including the digastric muscle and the mylohyoid muscle to stimulate the tongue base including the genioglossus muscle, the geniohyoid muscle, and the hyoglossus muscle; the second stimulation unit 220 attached to the upper part of the thyroid cartilage to stimulate the thyrohyoid muscle; and the third stimulation unit 230 attached to the inner part of the sternocleidomastoid muscle and to the lower part of the thyroid cartilage to stimulate the infrahyoid muscle including the sternohyoid muscle, the sternothyroid muscle, and the omohyoid muscle.

The first stimulation unit 210 includes electrodes that are respectively attached to both sides of the suprahyoid muscle at a predetermined distance and form a first channel, the second stimulation unit 220 includes electrodes that are respectively attached to both sides above the thyroid cartilage at a predetermined distance and form a second channel, and the third stimulation unit 230 includes electrodes that are respectively attached to both inner sides of the sternocleidomastoid muscle at a predetermined distance and form a third channel.

The stimulation units 200 include: the first stimulation unit 210 attached to one side of the bilateral suprahyoid muscle including the digastric muscle and the mylohyoid muscle to stimulate the tongue base including the genioglossus muscle, the geniohyoid muscle, and the hyoglossus muscle; the second stimulation unit 220 attached to the other side of the bilateral suprahyoid muscle including the digastric muscle and the mylohyoid muscle to stimulate the tongue base including the genioglossus muscle, the geniohyoid muscle, and the hyoglossus muscle; the third stimulation unit 230 attached to the upper part of the thyroid cartilage to stimulate the thyrohyoid muscle; and the fourth stimulation unit 240 attached to the inner part of the sternocleidomastoid muscle and to the lower part of the thyroid cartilage to stimulate the infrahyoid muscle including the sternohyoid muscle, the sternothyroid muscle, and the omohyoid muscle.

The first stimulation unit 210 includes electrodes that are attached to one side of the suprahyoid muscle at a predetermined distance and form a first channel, the second stimulation unit 220 includes electrodes that are attached to the other side of the suprahyoid muscle at a predetermined distance and form a second channel, the third stimulation unit 230 includes electrodes that are attached to both sides above the thyroid cartilage at a predetermined distance and form a third channel, and the fourth stimulation unit 240 includes electrodes that are attached to both inner sides of the sternocleidomastoid muscle at a predetermined distance and form a fourth channel.

Each stimulation unit 200 may have a frequency of 30 Hz to 100 Hz.

Meanwhile, muscles are classified into slow-twitch/red muscles (type I), which are endurance muscles, and fast-twitch/white muscles (type II), which are explosive muscles. Specifically, the type I muscles (slow-twitch/red muscles) contain more myoglobin than the type II muscles (fast-twitch/white muscles). As a result, the type I muscles have a relatively high muscular endurance, contract slowly and steadily, and accumulate less lactic acid and thus are highly resistant to fatigue.

Meanwhile, the type II muscles (fast-twitch/white muscles) produce a large amount of energy in a short time and are classified into type IIa and type IIb. The type IIb muscles have more fast-twitch characteristics and generate the greatest force. However, the type IIb muscles are the least efficient muscle fibers due to low oxidative capacity, high dependence on anaerobic metabolism, and high myosin ATPase activity. The type IIa muscles have characteristics of both fast-twitch and slow-twitch muscles and serve as intermediate muscle fibers between type I and types IIx and IIb.

Therefore, as the frequency of electrical stimulation increases, the proportion of highly fatigable muscle fibers (type IIb) among the fast-twitch muscles decreases and the proportion of less fatigable muscle fibers (type IIa) increases. Accordingly, the electrical stimulation therapy apparatus according to the present disclosure has therapeutic importance in sleep apnea by modifying the muscle fiber arrangement pattern.

The embodiment of the present disclosure can be embodied in a storage medium including instruction codes executable by a computer such as a program module executed by the computer. A computer-readable medium can be any usable medium which can be accessed by the computer and includes all volatile/non-volatile and removable/non-removable media. Further, the computer-readable medium may include all computer storage and. The computer storage media include all volatile/non-volatile and removable/non-removable media embodied by a certain method or technology for storing information such as computer-readable instruction code, a data structure, a program module or other data.

The method and system of the present disclosure have been explained in relation to a specific embodiment, but their components or a part or all of their operations can be embodied by using a computer system having general-purpose hardware architecture.

The above description of the present disclosure is provided for the purpose of illustration, and it would be understood by a person with ordinary skill in the art that various changes and modifications may be made without changing technical conception and essential features of the present disclosure. Thus, it is clear that the above-described examples are illustrative in all aspects and do not limit the present disclosure. For example, each component described to be of a single type can be implemented in a distributed manner. Likewise, components described to be distributed can be implemented in a combined manner.

The scope of the present disclosure is defined by the following claims rather than by the detailed description of the embodiment. It shall be understood that all modifications and embodiments conceived from the meaning and scope of the claims and their equivalents are included in the scope of the present disclosure.

### EXPLANATION OF CODES

100: Electrical stimulation therapy apparatus
120: Communication module
130: Processor
140: Memory
150: Input unit
200: Stimulation unit
210: First stimulation unit
220: Second stimulation unit
230: Third stimulation unit
240: Fourth stimulation unit

## Claims

1. An electrical stimulation therapy apparatus for sleep apnea, comprising:
a plurality of stimulation units attached to muscles involved in sleep apnea and configured with three or more channels; and
a processor which generates a control command to drive each of the stimulation units,
wherein the processor uses the stimulation units to sequentially stimulate a tongue base, a thyrohyoid muscle, and an infrahyoid muscle of a user according to a preset muscle activation sequence.

2. The electrical stimulation therapy apparatus of Claim 1,
wherein the stimulation units include:
a first stimulation unit attached to a suprahyoid muscle including a digastric muscle and a mylohyoid muscle to stimulate the tongue base including a genioglossus muscle, a geniohyoid muscle, and a hyoglossus muscle;
a second stimulation unit attached to an upper part of a thyroid cartilage to stimulate the thyrohyoid muscle; and
a third stimulation unit attached to an inner part of a sternocleidomastoid muscle and to a lower part of the thyroid cartilage to stimulate the infrahyoid muscle including a sternohyoid muscle, a sternothyroid muscle, and an omohyoid muscle.

3. The electrical stimulation therapy apparatus of Claim 2,
wherein the first stimulation unit includes electrodes that are respectively attached to both sides of the suprahyoid muscle at a predetermined distance and form a first channel,
the second stimulation unit includes electrodes that are respectively attached to both sides above the thyroid cartilage at a predetermined distance and form a second channel, and
the third stimulation unit includes electrodes that are respectively attached to both inner sides of the sternocleidomastoid muscle at a predetermined distance and form a third channel.

4. The electrical stimulation therapy apparatus of Claim 1,
wherein the stimulation units include:
a first stimulation unit attached to one side of a bilateral suprahyoid muscle including a digastric muscle and a mylohyoid muscle to stimulate the tongue base including a genioglossus muscle, a geniohyoid muscle, and a hyoglossus muscle;
a second stimulation unit attached to the other side of the bilateral suprahyoid muscle including the digastric muscle and the mylohyoid muscle to stimulate the tongue base including the genioglossus muscle, the geniohyoid muscle, and the hyoglossus muscle;
a third stimulation unit attached to an upper part of a thyroid cartilage to stimulate the thyrohyoid muscle; and
a fourth stimulation unit attached to an inner part of a sternocleidomastoid muscle and to a lower part of the thyroid cartilage to stimulate the infrahyoid muscle including a sternohyoid muscle, a sternothyroid muscle, and an omohyoid muscle.

5. The electrical stimulation therapy apparatus of Claim 4,
wherein the first stimulation unit includes electrodes that are attached to one side of the suprahyoid muscle at a predetermined distance and form a first channel,
the second stimulation unit includes electrodes that are attached to the other side of the suprahyoid muscle at a predetermined distance and form a second channel,
the third stimulation unit includes electrodes that are attached to both sides above the thyroid cartilage at a predetermined distance and form a third channel, and
the fourth stimulation unit includes electrodes that are attached to both inner sides of the sternocleidomastoid muscle at a predetermined distance and form a fourth channel.

6. The electrical stimulation therapy apparatus of Claim 1,
wherein each stimulation unit has a frequency of 30 Hz to 100 Hz.

7. An operation method of an electrical stimulation therapy apparatus for sleep apnea, comprising:
(a) attaching a plurality of stimulation units, which is configured with three or more channels, to muscles involved in sleep apnea; and
(b) sequentially stimulating a tongue base, a thyrohyoid muscle, and an infrahyoid muscle of a user according to a preset muscle activation sequence by using the stimulation units.

8. The operation method of an electrical stimulation therapy apparatus for sleep apnea of Claim 7,
wherein the stimulation units include:
a first stimulation unit attached to a suprahyoid muscle including a digastric muscle and a mylohyoid muscle to stimulate the tongue base including a genioglossus muscle, a geniohyoid muscle, and a hyoglossus muscle;
a second stimulation unit attached to an upper part of a thyroid cartilage to stimulate the thyrohyoid muscle; and
a third stimulation unit attached to an inner part of a sternocleidomastoid muscle and to a lower part of the thyroid cartilage to stimulate the infrahyoid muscle including a sternohyoid muscle, a sternothyroid muscle, and an omohyoid muscle.

9. The operation method of an electrical stimulation therapy apparatus for sleep apnea of Claim 8,
wherein the first stimulation unit includes electrodes that are respectively attached to both sides of the suprahyoid muscle at a predetermined distance and form a first channel,
the second stimulation unit includes electrodes that are respectively attached to both sides above the thyroid cartilage at a predetermined distance and form a second channel, and
the third stimulation unit includes electrodes that are respectively attached to both inner sides of the sternocleidomastoid muscle at a predetermined distance and form a third channel 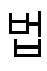 .

10. The operation method of an electrical stimulation therapy apparatus for sleep apnea of Claim 7,
wherein the stimulation units include:
a first stimulation unit attached to one side of a bilateral suprahyoid muscle including a digastric muscle and a mylohyoid muscle to stimulate the tongue base including a genioglossus muscle, a geniohyoid muscle, and a hyoglossus muscle;
a second stimulation unit attached to the other side of the bilateral suprahyoid muscle including the digastric muscle and the mylohyoid muscle to stimulate the tongue base including the genioglossus muscle, the geniohyoid muscle, and the hyoglossus muscle;
a third stimulation unit attached to an upper part of a thyroid cartilage to stimulate the thyrohyoid muscle; and
a fourth stimulation unit attached to an inner part of a sternocleidomastoid muscle and to a lower part of the thyroid cartilage to stimulate the infrahyoid muscle including a sternohyoid muscle, a sternothyroid muscle, and an omohyoid muscle.

11. The operation method of an electrical stimulation therapy apparatus for sleep apnea of Claim 10,
Wherein the first stimulation unit includes electrodes that are attached to one side of the suprahyoid muscle at a predetermined distance and form a first channel,
the second stimulation unit includes electrodes that are attached to the other side of the suprahyoid muscle at a predetermined distance and form a second channel,
the third stimulation unit includes electrodes that are attached to both sides above the thyroid cartilage at a predetermined distance and form a third channel, and
the fourth stimulation unit includes electrodes that are attached to both inner sides of the sternocleidomastoid muscle at a predetermined distance and form a fourth channel.

12. The operation method of an electrical stimulation therapy apparatus for sleep apnea of Claim 7,
wherein each stimulation unit has a frequency of 30 Hz to 100 Hz.
